Europäisches Patentamt

European Patent Office

Office européen des brevets

(11) Publication number: **0 305 275**
**A2**

(12) # EUROPEAN PATENT APPLICATION

(21) Application number: 88402115.5

(22) Date of filing: 16.08.88

(51) Int. Cl.⁴: **C 12 N 5/00**
A 01 H 1/00, C 12 N 15/00,
A 01 N 63/00, C 07 K 13/00,
C 12 N 1/20

(30) Priority: 17.08.87 GB 8719414
29.12.87 GB 8730261

(43) Date of publication of application:
01.03.89 Bulletin 89/09

(84) Designated Contracting States:
AT BE CH DE ES FR GB GR IT LI LU NL SE

(71) Applicant: **PLANT GENETIC SYSTEMS N.V.**
**Kunstlaan Avenue des Arts, 46**
**B-1040 Bruxelles (BE)**

(72) Inventor: **Vaeck, Mark**
**Aarschotsebaan, 4**
**B-2959 Elewijt (BE)**

**Hofte, Hermanus**
**Savaanstraat 102**
**B-9000 Gent (BE)**

**Botterman, Johan**
**Het Wijngaardeke 5**
**B-9721 Zevergen-De Pinte (BE)**

(74) Representative: **Gutmann, Ernest et al**
**S.C. Ernest Gutmann - Yves Plasseraud 67, boulevard**
**Haussmann**
**F-75008 Paris (FR)**

The microorganism(s) has (have) been deposited with Deutsche Sammlung von Mikroorganismen under number(s) 4752 - 4753.

(54) **Plants transformed with a DNA sequence from Bacillus thuringiensis.**

(57) A plant cell transformed with a Bacillus thuringiensis gene coding for a protein of at least 66 kDa that is toxic to Coleoptera. A plant containing the transformed cell is resistant to Coleoptera.

EP 0 305 275 A2

## Description

## PLANTS TRANSFORMED WITH A DNA SEQUENCE FROM BACILLUS THURINGIENSIS

Background of the Invention

This invention relates to a DNA sequence (the " bt13 gene") which has been found independently in the genome of both the strains Bacillus thuringiensis tenebrionis (U.K. patent application 87.19414) and Bacillus thuringiensis S1 (U.K. patent application 87.30261). The bt13 gene encodes a 66 kDa crystal protein (the "Bt13 protein") which is believed to be the active protein in the crystal toxin (the "Bt13 toxin") produced by these B.t. strains. The Bt13 toxin is toxic to Coleoptera (beetles).

This invention particularly relates to a plant, such as a tomato or potato plant, the genome of which is transformed with the bt13 gene so that some or all of the cells of the plant produce the Bt13 protein, thereby rendering the plant resistant to Coleoptera.

The bt13 gene has been found to be identical in nucleotide sequence to: 1) the B.t. gene that is disclosed in European patent publication 0,213,818 (1987) and that codes for a Coleoptera toxin containing a 86 kDa protein; and 2) the gene found in the B. t. strain which is disclosed in European patent publication 0,149,162 (1985) and which produces a Coleoptera toxin containing proteins of 20, 40, 65 and 70 kDa.

Summary of the Invention

In accordance with this invention, a plant cell genome is transformed with all part of the bt13 gene, particularly the part of the bt13 gene coding for the Bt13 protein. The resulting plant cell can be used to produce a plant, in which some or all of the plant cells express the gene by producing the Bt13 protein, thereby rendering the plant Coleoptera resistant.

Also in accordance with this invention, a method is provided for transforming the plant cell genome with all or part of the bt13 gene.

Further in accordance with this invention, a DNA sequence is provided that can be obtained from the bt13 gene and that codes just for the Bt13 protein. A plant cell, transformed with this DNA sequence can produce a Coleoptera toxin that consists essentially of the Bt13 protein.

Detailed Description of the Invention

In accordance with this invention, the bt13 gene can be isolated in a conventional manner from the B. t. strains deposited at the Deutsche Sammlung Von Mikroorganismen ("DSM") at Göttingen, Federal Republic of Germany: 1) under accession number 4288 on October 22, 1987 (as described in U.K. patent application 87.30261); and 2) under accession number 2803 (as described in European patent publication 0,149,162). In this regard, the DNA sequence of the bt13 gene can be deduced from the amino acid sequence of the Bt13 protein that is produced by the deposited B. t. strains and is toxic to Coleoptera.

The bt13 gene can also be stably inserted in a conventional manner into the nuclear genome of a single plant cell, and the so-transformed plant cell be used to produce a transformed plant in a conventional manner. In this regard, a Ti-plasmid, containing the bt13 gene, in Agrobacterium tumefaciens can be used to transform the plant cell, using the procedures described, for example, in European patent application 0,116,718, PCT publication WO 84/02913 and European patent application 87/400,544.0 (which are incorporated herein by reference).

Preferably, the bt13 gene is inserted in a plant genome downstream of, and under the control of, a promoter which directs the expression of the gene in the plant cell. Preferred promoters include the strong constitutive exogenous plant promoters such as: the promoter from cauliflower mosaic virus directing the 35S transcript (Odell, J.T., Nagy, J. and Chua, N.M. (1985) Nature 313 810-812) [the "35S promoter"]; the 35S promoter from the CaMV isolate Cabb-JI (Hull and Howell (1987) Virology 86, 482-493) [the "35S3 promoter"]; and the TR1' promoter and the TR2' promoter which drive the expression of the 1' and 2' genes, respectively, of the T-DNA (Velten et al (1984) EMBO J. 3, 2723-2730) [the "TR1' promoter" and "TR2' promoter", respectively]. Alternatively, a promoter can be utilized which is not constitutive but rather is specific for one or more tissues or organs of the plant (e.g., leaves and/or roots) whereby the Bt13 protein is expressed only in cells of the specific tissue(s) or organ(s). For example, the bt13 gene could be selectively expressed in the leaves of a plant (e.g., tomato or potato) by placing the gene under the control of a light-inducible promoter such as the promoter of the ribulose-1,5-biphosphate carboxylase small subunit gene of the plant itself or of another plant such as pea as disclosed in U.S. patent application 821,582, filed January 22, 1986 (which is also incorporated herein by reference). Another alternative is to use a promoter whose expression is inducible, (e.g., by temperature or chemical factors).

It is further preferred that the bt13 gene be inserted in the plant genome upstream of suitable polyadenylation and transcription termination signals such as that of: the octopine synthase gene (Gielen et al. (1984) EMBO J. 3, 835-845); or the T-DNA gene 7 (Velten and Schell (1985) Nucleic Acid Research ("NAR") 13, 6981-6998).

It is further preferred that the bt13 gene be in the same transcriptional unit as, and under the control of, the same promoter as a selectable marker gene whereby the two genes are expressed in a transformed plant as a fusion protein (U.S. patent application 821,582, filed January 22, 1986; Vaeck et al (1987) Nature 327, 33-37) . Any conventional marker gene can be utilized, the expression of which can be used to select transformed plant cells. An example of a suitable selectable marker gene is an antibiotic resistance gene such as the neo gene coding for kanamycin resistance (European patent application 87/400,544.0; U.S. patent application 821,582).

It is still further preferred that the bt13 gene be inserted in the plant cell genome, together with a gene, such as the bt2 gene (U.S. patent application 821,582; Höfte et al (1986) Eur. J. Biochem. 161, 273-280), which codes for a toxin against Lepidoptera. Thereby, a transformed plant can be produced which is resistant to both Lepidoptera and Coleoptera.

The following Examples illustrate the invention. The Figures referred to in the Examples, are as follows:

Fig. 1 shows the restriction map of the 2.9 kb HindIII fragment of the plasmids pBt130 and pBt130′ containing the bt13 gene in Examples 1 and 2.

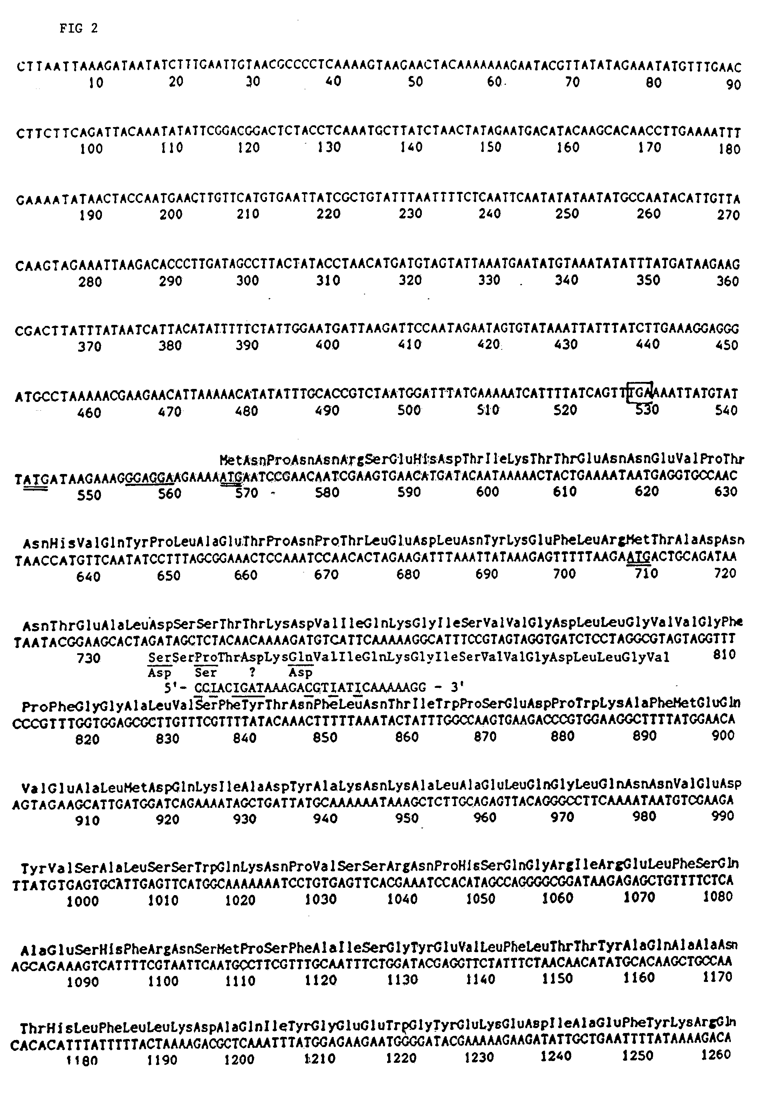

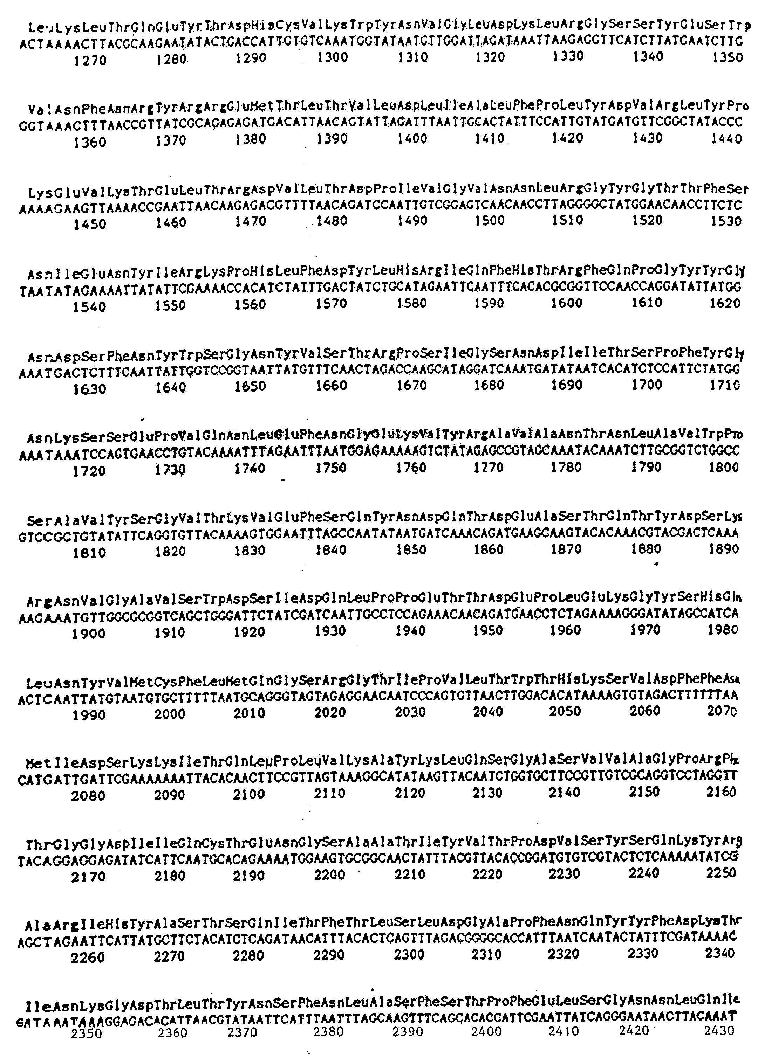

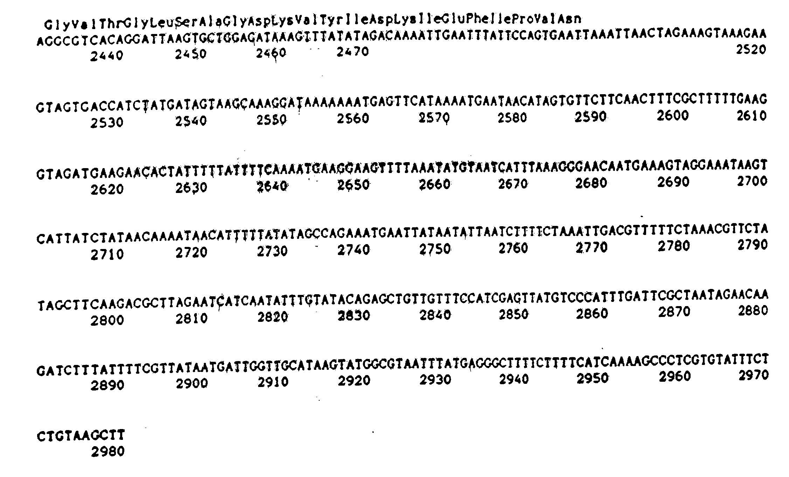

Fig. 2 shows the nucleotide sequence, including an open reading frame ("ORF"), of the bt13 gene and the deduced amino acid sequence of the Bt13 protein. The bt13 gene has the nucleotide sequence shown in Fig. 2 from nucleotide 566 to nucleotide 2497. A synthetic DNA probe, used for screening cosmid clones, is also indicated, and the mismatches with the bt13 gene's DNA sequence are underlined. The 22 N-terminal amino acids of the Bt13 protein are indicated. The question mark ("?") indicates that this amino acid residue was not unambigously determined, and two possible amino acid residues are shown. The in-frame ATG-codons of the bt13 gene are underlined twice. The first in-frame upstream stopcodon is boxed, and the putative Shine-Dalgarno sequence is underlined.

Fig. 3 shows the 3′ end sequence of the bt13 gene with the predicted amino acid sequence of the Bt13 protein. The homologous sequences in other known B.t. toxins are indicated. The arrows indicate the C-terminus of the largest non-toxic fragment (the "fragment 1") and the smallest toxic fragment (the "fragment 2") of the Bt2 toxin (U.S. patent application 821,582; Höfte et al (1986)). The numbering in this figure does not correspond to the numbering of the sequence shown in Fig. 2.

Fig. 4 shows schematically the cloning of the 5′ end (fragment 1) and the 3′ end (fragment 2) of the bt13 gene into the plasmid pMA5-8.

Fig. 5 shows schematically the mutagenesis of the 5′ and the 3′ end of the bt13 gene. The nucleotide sequence changes are boxed. The codons of the bt13 gene are numbered.

Fig. 6 shows schematically: A) the cloning of the modified 3′ end of the bt13 gene into the plasmid pUC19 to form the vector pBt136 and the arrangement of the inserted fragment in pBt136; and B) the construction of E. coli expression vectors pBt134 and pBt135 from pBt136.

Fig. 7 shows schematically the construction of a plasmid pBt13neo of fused genes and the sequence at the junction of the fused genes.

Fig. 8 shows schematically the construction of plant expression vectors containing all or part of the bt-13 gene: A) pGSJ141 and pGSJ142; B) pGSJ143 and pGSJ144; C) pGSJ147 and pGSJ148; and D) pTVE38.

Fig. 9 shows schematically the construction of plant expression vectors PGSJ145 and pGSJ146 containing the bt13 gene.

Unless otherwise stated in the Examples, all procedures for making and manipulating recombinant DNA were carried out by the standardized procedures described in Maniatis et al, Molecular Cloning - A Laboratory Manual, Cold Spring Harbor Laboratory (1982). E. coli containing the following plasmids and vectors, used in the Examples, have been deposited in the DSM under the provisions of the Budapest Treaty:

| Plasmid or vector | DSM Accession No. | Date |
|---|---|---|
| pbt13-neo | 4752 | August 3, 1988 |
| pGSH150 | 4753 | August 3, 1988 |

Example 1 - Isolating the bt13 gene

A polyclonal antiserum was raised in mouse against total Bacillus thuringiensis tenebrionis crystal toxin solubilized in a buffer solution containing 50 mM Na2CO3, pH 10 + 10 mM dithiothreitol ("DTT"). The crystal toxin was purified according to Mahillon, J., and Delcour, J. (1984) J. Microbiol. Methods 3, 69-73. The crystal toxin contained one major 66 kDa protein as shown in SDS-PAGE (Laemmli, U. (1970) Nature 227, 680-685). The N-terminal sequence of the major 66 kDa protein in the crystals was determined. To this end, total crystal proteins were blotted on polybrene coated glass fibers (European patent application 86/401933.6), and the 66 kDa protein was extracted for amino acid sequencing. The sequence of the first 22 amino acids, as shown in Fig. 2, were determined by gas phase sequencing using a gas phase sequenator (Applied Biosystems Inc. USA) operated as described by Hewick, R.M., Hunkapillar, M.W., Hood, L.E. and Dreyer, W.J. (1981) J. Biol. Chem. 256, 7990-7997. Purified solubilized crystals showed a LD50 of < 100 ng/cm$^2$ against the Colorado potato beetle (Leptinotarsa decemlineata).

A library of partial Sau3A-digested total DNA of B. t. tenebrionis was constructed in the cosmid vector pLAFB1 which is a derivative of pLAFR1 (Friedman, A.M., Long, S.R., Brown, S.E., Biukema, W.J. and Ansubel, F.M. (1982) Gene 18, 289-296) with a BamHI-linker in the former unique EcoR1 site. The polyclonal mouse antiserum, raised against the crystal toxin of B. t. tenebrionis, was used to screen approximately 2000 clones in a colony immuno assay. Twenty-one positive clones were further analysed by Western blotting (Towbin, H., Stähelin, T. and Gordon, J. (1979) Proc. Natl. Acad. Sci. USA ("PNAS") 76, 4350-4353) of total cellular extracts. Among these clones, four different types of clones could be distinguished based upon the visualized cross-reacting proteins. The four types of clones are set forth in the following Table 1.

## Table 1: types of E. coli clones expressing proteins that cross-react with B.t. tenebrionis crystal antiserum

| Type | Example of clone | Size of cross-reacting proteins (kDa) | hybridization to a synthetic oligonucleotide (29-mer) |
|------|------------------|---------------------------------------|--------------------------------------------------------|
| I | pBTT16 | 66 | - |
| II | pBTT18 | 66 + 71 (broad band) | - |
| III | pBTT32 | 66 + 71 (fine band) | + |
| IV | pBTT30 | larger strongly cross reacting protein | - |

A 29 bp degenerated DNA probe was synthesized based upon a sequence of 10 amino acids (Fig. 2) of the B.t.tenebrionis crystal toxin using the procedure of Itekaru et al (1984) Ann. Rev. of Biochem. 53, 323-356. Representatives of the four types of cosmid clones were hybridized at low stringency reaction conditions.

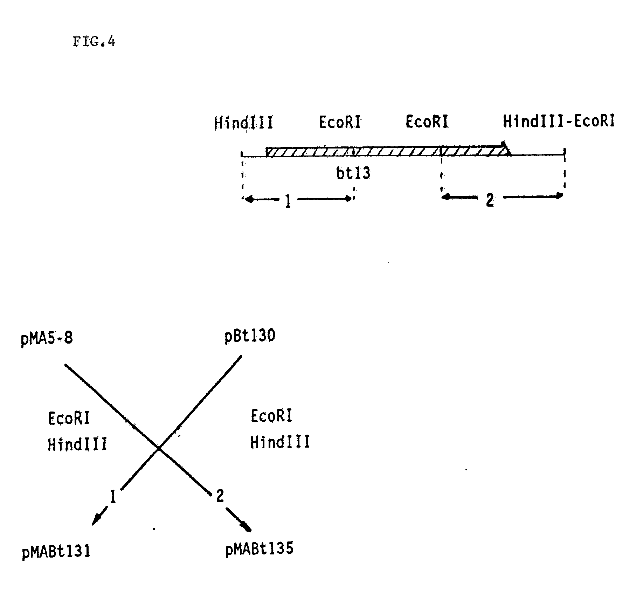

Only pBTT32 (Type III) showed homology with the probe. The hybridizing sequence was localized on a 1.6 kb EcoRI- HindIII fragment. DNA sequencing (Maxam and Gilbert (1980) Methods In Enzymology 65, 499-560) from both sides of the fragment revealed that an open reading frame, showing some homology to the bt2 gene (Höfte et al (1986), could be localized on a 2.9 kb HindIII fragment. This fragment was subcloned in pUC8 (Vieira, J. and Messing, J. (1982) Gene 19, 259-268) giving rise to plasmid pBt130 (Fig. 4).

pBt130 directed the synthesis in E. coli of a 71 kDa protein which reacted with the polyclonal mouse anti-B.t. tenebrionis crystal toxin serum as shown in a Western blot. This 71 kDa protein was processed in E. coli into a protein of the same size as the major 66 kDa crystal protein of B. t. tenebrionis. pBt130 contained the bt13 gene in the opposite orientation with respect to the promoter (pLac) of the vector. Therefore, the gene was transcribed from its own promoter. To enhance its expression, the bt13 gene was cloned, as a HindIII fragment behind the lambda P$_L$-promoter in plasmid pLK59 (Botterman, J. and Zabeau, M. (1987) DNA 6, 583-591) which was then used to transform the E. coli strain K-12ΔH1Δtrp carrying a temperature sensitive repressor of the lambda P$_L$ promoter (Bernard et al (1979) Gene 5, 59-76). This construction provided a high level of expression of the Bt13 protein upon temperature induction at 42°C of the transformed E. coli K-12ΔH1Δtrp.

Example 2 - Isolating another bt13 gene (the "bt13' gene")

The crystal toxin of the B.t. strain, deposited at the DSM under accession no. 4288, was purified using the procedure described by Krieg, V.A., Huger, A.M., Langenbruch, G.A. and Schnetter, W. (1983) Z. Angew. Entomology 96, 500-508. A polyclonal antiserum was raised in rabbit (U.S. patent application 821,582) against the crystal toxin produced by the B.t. strain DSM 4288. This antiserum was used to detect the crystal protein produced by this B.t. strain by Western blotting.

The 66 kDa crystal protein was shown by SDS-page (Laemmli, U. (1970)) to be the major component of the crystal toxin produced by the B.t. strain DSM 4288. The N-terminal sequence of the 66 kDa protein in the toxin was then determined. To this end, total crystal proteins were blotted on polybrene-coated glass fibers (European patent application 86/401933.6), and an approximately 66 kDa fragment was extracted for amino acid sequencing by gas phase sequencing (Hewick et al (1981)).

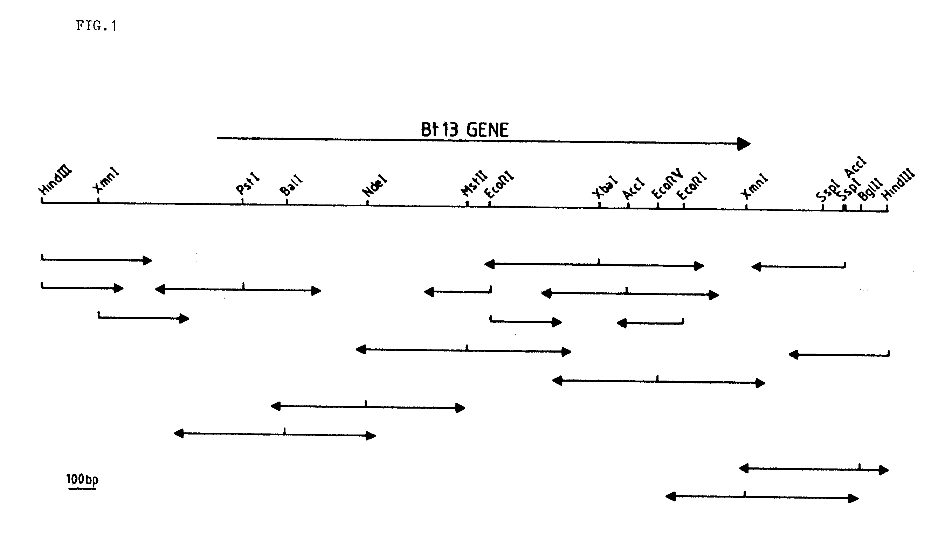

A library of partial Sau3A-digested total DNA of the strain was constructed in the plasmid pUC8 (Viera and Messing (1982)) using the BamHI site. An approximately 1000 basepairs Ball-Xbal fragment (Fig. 1) derived from the bt13 gene of Example 1 was radioactively ($^{32}$P) labelled and used as a probe to screen the library. Plasmid DNA was extracted from strongly hybridizing clones, digested with HindIII and analyzed in Southern blotting with the probe derived from the bt13 gene. Several plasmids were identified, containing a 2.9 kb HindIII fragment that hybridized with the probe. DNA sequencing (Maxam and Gilbert (1980)) from both sides of the HindIII fragment revealed that an ORF, showing homology to the bt13 gene of Example 1, was localized on this HindIII fragment. This fragment was subcloned in pUC8 (Viera and Messing (1982)), giving rise to plasmid pBt130'.

pBt130' directed the synthesis in E. coli of a 71 kDa protein which reacted with the polyclonal rabbit anti-serum to the crystal toxin of B.t. strain DSM 4288 in Western blot. The 71 kDa protein was processed in E. coli into a protein of the same size as the major 66 kDa crystal protein of B.t. strain DSM 4288. pBt130' contained the bt13' gene in the opposite orientation with respect to the promoter (pLac) of the vector. Therefore, the gene was transcribed from its own promoter. To enhance the expression thereof, the bt13' gene was cloned as a HindIII-fragment behind the lambda $P_L$-promoter in plasmid pLK59 (Botterman and Zabeau (1987)), which was then used to transform the E. coli strain K-12ΔH1Δtrp (Bernard et al (1979)). This construction provided a high level of expression of the bt13' gene upon temperature induction at 42°C of the transformed E. coli K-12ΔH1Δtrp.

Example 3 - Comparing of the structure of the bt13 gene and the bt13' gene

It was found that the bt13 gene of Example 1 and the bt13' gene of Example 2 are identical despite the fact that they were independently isolated from two strains discovered in different areas. Both bt13 genes encode a polypeptide of 644 amino acids with a predicted molecular weight of 71 kDa. The ATG initiator codon in both bt13 genes is localized at position 566 in the DNA sequence of the genes (Fig. 2). This conclusion was based upon the following assumptions:

A) The N-terminal sequence of the major 66 kDa crystal protein coincides with the predicted amino acid sequence from both bt13 genes starting at position 737 (Fig. 2). This indicates that the 66 kDa protein is a processed product of a longer 71 kDa precursor. Both the 66 and 71 kDa proteins were also observed in the E. coli clones expressing the bt13 gene in Examples 1 and 2.

B) The first in-frame stopcodon upstream of bp 737 is at position 527. In between, there are three in-frame ATG-codons. The ATG at position 566 is a very likely candidate for an initiator codon:

1) It is the only in-frame ATG codon preceded by a clear Shine and Dalgarno sequence (Shine, J. and Dalgarno, L. (1974) PNAS 71, 1342-1346).

2) The predicted N-terminal sequence starting at this position shows homology to the N-terminus of the bt13 gene:M - - N P N N
the bt2 gene:M D N N P N

C) The ORF, starting at position 566, ends at position 2497.

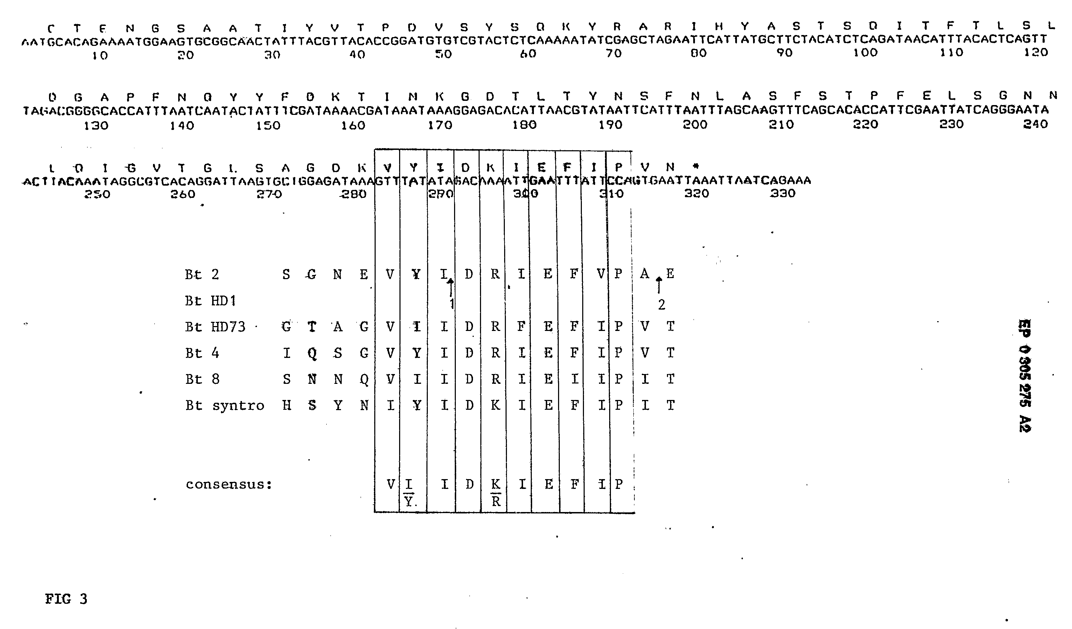

D) The 66kDa proteins, coded by both bt13 genes, show (in Fig. 3) local stretches of homology with the Bt2 and Bt8 crystal toxins produced by the B.t. berliner 1715 strain (Höfte et al (1986)) and the B. t. israelensis 4Q-272 strain (US application serial No. 021,405, filed March 4, 1987; Chungjatupornchai et al (1988) Eur. J. Biochem. 173, 9-16), respectively. The C-terminal sequence of each of the bt13 genes also shows a strong homology with known sequences in other B.t. toxins (Fig. 3). Moreover, the C-terminus of the smallest toxic fragment of the Bt2 crystal toxin (Vaeck et al (1987)), as determined by deletion mapping, coincides with the amino acid sequence preceding the C-terminal amino acid of the crystal proteins of each of the bt13 genes. The Bt13 protein can thus be considered as a naturally occurring truncated active toxin.

These assumptions have been confirmed by McPherson et al (1988) Bio/Technology 6, 61-66.

Example 4 - Producing Bt13 protein in E.coli

Using the procedure of Höfte et al (1986), a purified Bt13 protein (66 kDa), together with its 71 kDa precursor, was produced from induced cultures of the transformed E. coli K-12ΔH$_1$Δtrp of Examples 1 and 2.

The Bt13 protein, so produced, showed an LD50 of 12 ng/cm$^2$ against Leptinotarsa decemlineata.

Example 5 - Constructing plant transformation cassettes with the bt13 gene

In order to obtain optimal expression of the bt13 gene in transgenic plants, chimeric constructs were made as shown in Figs. 4 to 9 with different strong transcription initiation signals (promoters), using each of the bt13 genes of Examples 1 and 2. Since the bt13 gene in the E. coli K-12ΔH1Δtrp strain of Examples 1 and 2 was not readily available as a gene cassette, the gene was modified as follows.

A BamHI site was created abutting the ATG codon of the bt13 gene. The 4th nucleotide of the bt13 gene was changed from an A to a G by site directed mutagenesis yielding an unique BamHI site in the gene by the procedure described in the article by Stanssens, P., McKeown, Y., Friedrich, K. and Fritz, H.J. (1987), entitled "Oligonucleotide-directed construction of mutations by the gapped duplex DNA method using the pMa/c phasmid vectors", published in the Collection of experimental procedures distributed at the EMBO course entitled "Directed mutagenesis and protein engineering" in July 1987 at the Max Planck Institut für Biochemie, Martinsried, Federal Republic of Germany. According to the "Kozak-rules" (Kozak, M. (1986) Cell 44, 283-292), this mutation should also optimize the translation initiation in plant cells. However, this mutation also changes the 2nd amino acid of the bt13 gene from Asn to Asp. The effect of this mutation on the activity of the Bt13 protein was assessed in a toxicity assay after expression of the modified protein in E. coli.

In order to avoid a change of the second codon of the bt13 gene, an alternative construction was made. A Hinf1 site was provided directly downstream of the ATG-initiator codon ('AT G AAT C C') to permit the direct fusion of the second codon to the initiator present in plant promoter fragments.

Further constructions carrying the fragment encoding the processed 66 kDa protein were made. It is believed that the Bt13 protein is synthesized as a precursor of about 71 kDa that is processed into the 66 kDa Bt13 protein in E. coli and in B.t. strains (during the crystal formation). Unlike other B.t. toxins (Höfte et al (August 1988) Applied and Environmental Microbiology, in press), the 66 kDa Bt13 protein is present in the processed form already in the crystals of B. thuringiensis tenebrionis. Because it is unknown if the 71 kDa precursor protein would be correctly activated in the midgut of Coleoptera larvae or in plant cells, an alternative approach was to express the gene fragment encoding the processed 66 kDa protein, as such, in transformed plants. To this end, a restriction site was created at the putative processing site of the amino acid at position 58 of the precursor protein (Fig. 2).

A FokI site was introduced 9 basepairs upstream of the first codon of the gene fragment encoding the 66kDa protein by the mutagenesis procedure of Stanssens et al (1987). This yielded a modified bt13 gene called "bt13-57". The recognition sequence of FokI was separated from its cleavage site which allowed it to generate restriction fragments exactly at the processing site.

To allow proper fusion of the bt13 gene coding region to transcription termination and polyadenylation signals in plant expression vectors, the non-coding sequence downstream (3' end) in the bt13 gene was removed. To this end, an oligonucleotide with an EcoRI site, overlapping the TAA stopcodon, was introduced into the bt13 gene by the mutagenesis procedure of Stanssens et al (1987).

In order to be able to select easily transgenic plants producing sufficient Bt13 protein to kill Coleoptera, hybrid gene constructions were made with a gene encoding a selectable marker as described in U.S. patent application 821,582 and Vaeck et al (1987). Such a hybrid construction allows the selection of transformants whose marker gene expression is sufficiently great that one can suitably predict that the bt13 gene expression also is sufficiently great to render the transformants Coleoptera resistant (Höfte et al (1988) FEBS Letters 226, 364-370). For this purpose, bt13-neo hybrid gene fusions were constructed. The oligonucleotide with the EcoRI site, from the preceding paragraph, was used to generate a HpaI site in front of the stopcodon. This site allowed the codon 643 of the bt13 gene and of the bt13-57 gene to be fused to a neo gene fragment encoding an active neomycin phosphotransferase enzyme II ("NPTII") as described in U.S. patent application 821,582. The C-terminal Asn was not believed to be essential for toxicity. The resulting fusion proteins were produced in E. coli transformed with the bt13-neo hybrid gene fusions by the procedure described by Höfte et al (1986) and tested for Bt13 and NPTII activity as described below.

To evaluate the effect of the modifications, described above, on the activity of the Bt13 protein, the modified bt13 genes were expressed in E. coli and their modified Bt13 protein products were tested against the Colorado potato beetle. To this end, the bt13 gene fragments were fused in phase to the ATG-initiator codon of the cro gene of phage lambda under control of the $P_R$ promotor of phage lambda (Botterman and Zabeau (1987)).

As described below, the expression of the bt13 genes in plants was obtained by placing the different bt13 cassettes, described above, under the control of a 35S, a TR1' or a TR2' promoter at the 5' end. Polyadenylation and transcription termination signals from the octopine synthase gene ("ocs") (Gielen et al (1984)) or from the T-DNA gene 7 ("g7") (Velten and Schell (1985)) were also provided at the 3' end of the gene cassettes. Except for the hybrid gene contructions, the cassettes further contained a selection marker for transformants, i.e., the neo gene, controlled by its own TR1' promoter or promoter from the nopaline synthase gene ("Pnos" -- U.S. patent application 821,582) and its own OCS polyadenylation and transcription termination signals. To generate plants protected against both Lepidoptera and Coleoptera, constructs containing both the bt2 and bt13 genes were also made. Each of these chimeric constructs was inserted in a plant transformation vector of a disarmed Ti plasmid derived from Agrobacterium tumefaciens (Deblaere et al (1985) NAR 13, 4777-4788). The plant expression constructs, that were made, are summarized in the following Table 2.

## Table 2 : Plant expression constructs containing a bt13 gene

| 5' end | Bt gene | 3' end | Chimeric selectable marker gene | Plant Expression Construct |
|--------|---------|--------|--------------------------------|----------------------------|
| TR2' | btneo860 | g7 | | pGSH152 |
| TR2' | bt884 | g7 | | pGSH163 |
| 35S | bt13 | g7 | Pnos-neo-3'ocs | pGSJ141 |
| TR2' | bt13 | g7 | TR1'-neo-3'ocs | pGSJ142 |
| 35S | bt13-57 | g7 | Pnos-neo-3'ocs | pGSJ143 |
| TR2' | bt13-57 | g7 | TR1'-neo-3'ocs | pGSJ144 |
| TR1' | bt13 | ocs | | pGSJ145 |
| 35S | bt13 | ocs | TR1'-neo-3'ocs | pGSJ146 |
| TR2' | bt13-neo | g7 | | pGSJ147 |
| 35S | bt13-neo | g7 | | pGSJ148 |
| 35S3 | bt13-neo | g7 | | pTVE38 |
| 35S3 | bt13-57-neo | g7 | | pTVE39 |

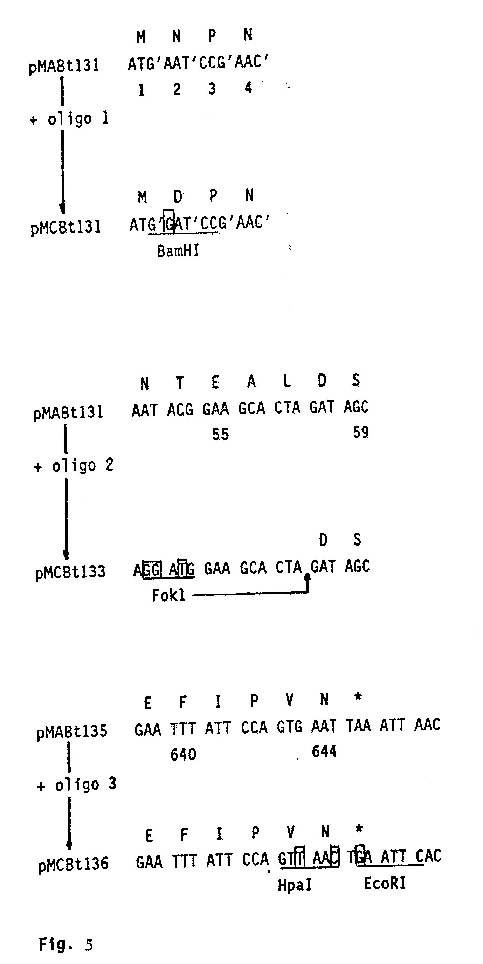

For the construction of the bt13 gene cassettes, the restriction sites - BamHI, FokI, HpaI and EcoRI -- were created in the bt13 gene coding region using site-directed mutagenesis with oligonucleotides as mutagenic primers (Stanssens et al (1987)). The vectors described by Stanssens et al (987) were used. In this regard, the EcoRI-HindIII fragments respectively containing the 5′ end (fragment 1) and 3′ end (fragment 2) of the bt13 gene were isolated from pBt130 and cloned into pMA5-8 (On deposit at the DSM under accession number 4567), giving rise to pMABt131 and pMABt135, respectively (Fig. 4). Three oligonucleotides were then synthesized : 1) 21-mer : 5′-GGAAGAAAAAT GGATCC AAC-3′; 2) 25-mer: 5′-GCAGATAATA GGAAG CAATCAG-3′; and 3) 34-mer: 5′-CTTTCTAGT GAATTC A GTTAA CTGGAATAAATTC; using the procedure described by Itekaru et al (1984). Using the gapped duplex procedure of Stanssens et al (1987), oligonucleotides 1) and 2) were used to introduce respectively a BamHI and FokI site in the N-terminal sequences of the bt13 gene, and oligonucleotide 3) was used to create a HpaI and EcoRI site at the C-terminus of the gene. This yielded the respective plasmids pMCBt131, pMCBt133 and pMCBt136 (Fig. 5).

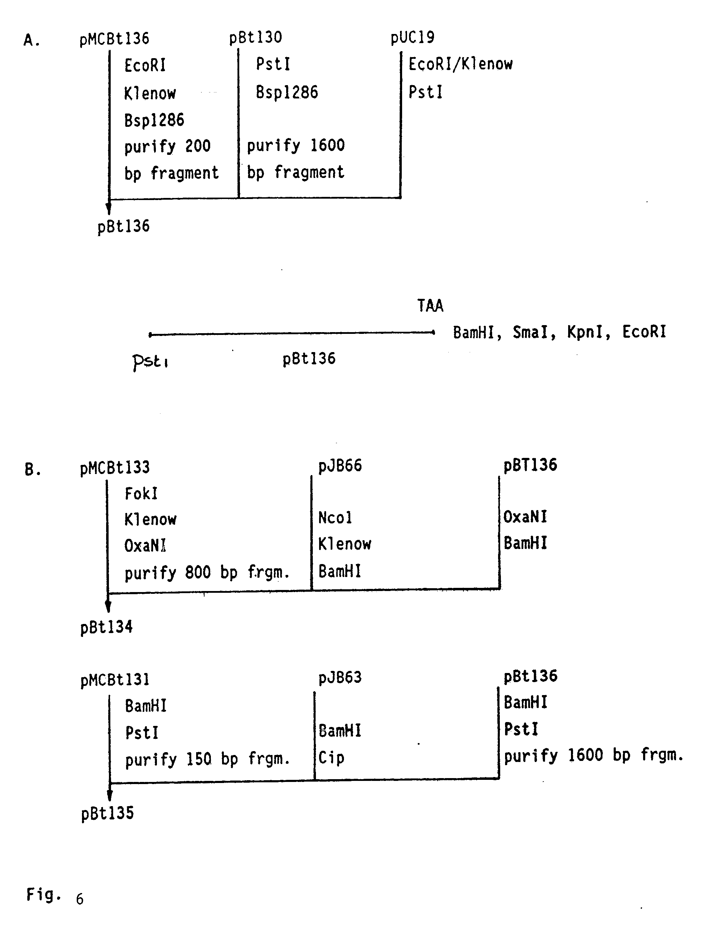

To facilitate the reconstruction of the bt13 gene, fragments from pMCBt136 and pBt130 were cloned in pUC19 (Yanish-Perron, C., Viera, J. and Messing, J. (1985) Gene 33, 103-119), yielding pBt136 as shown in Fig. 6A. In this way a unique BamHI site was introduced immediately downstream of the gene, and the fragment, containing the C-terminal end of the bt13 gene, could be used for reconstruction of the entire bt13 gene carrying the described mutations at the N-terminus.

As shown in Fig. 6B, the N-terminal fragments from the bt13 gene were retrieved from pMCBt133 as a FokI-OxaNI fragment and from pMCBt131 as a BamHI-PstI fragment. These N-terminal fragments and suitable C-terminal fragments from pBt136 were ligated in the E. coli expression vectors pJB66 and pJB63, respectively (Botterman and Zabeau (1987)), each carrying a bacteriophage lambda $P_R$ promoter and the ribosome binding site of the cro gene of phage lambda. This yielded pBt134 and pBt135, respectively.

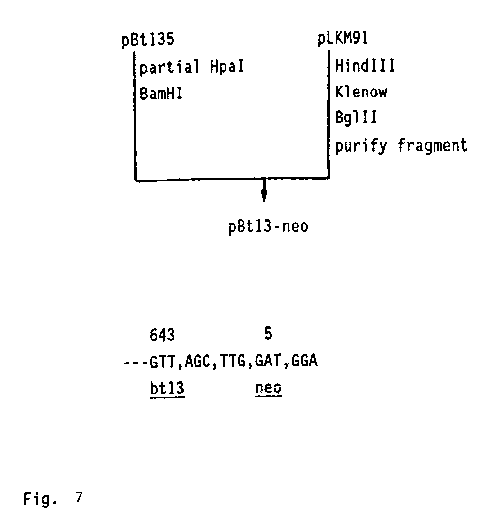

Making use of the HpaI site present at the 3′ end of the bt13 gene in pBt135, the bt13 gene was fused in phase to a truncated neo gene encoding an active C-terminal fragment (Reiss, B., Sprengel, R., Will, H. and Schaller, H. (1984) Gene 30, 217-223). The neo gene fragment was obtained from pLKM91 (Botterman (1986)), yielding pBt13-neo as shown in Fig.7.

The plasmids pBt134, pBt135 and pBt13-neo, carrying a regulatable strong $P_R$ promoter, were used to transform E. coli strain K-12ΔH₁Δtrp. The transformed E. coli was then temperature induced to drive expression of the different gene products as described by Botterman and Zabeau (1987). The impact of the gene mutations upon the activity of the gene products was analysed. The Bt13-NPTII fusion proteins showed NPTII activity without adversely affecting the LD50 activity that was observed above with the intact Bt13 protein.

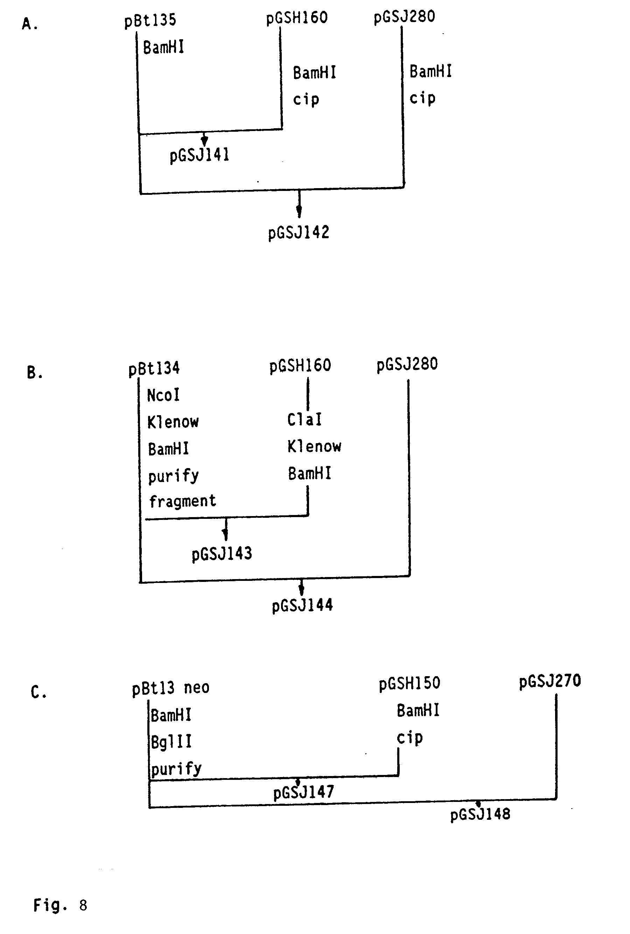

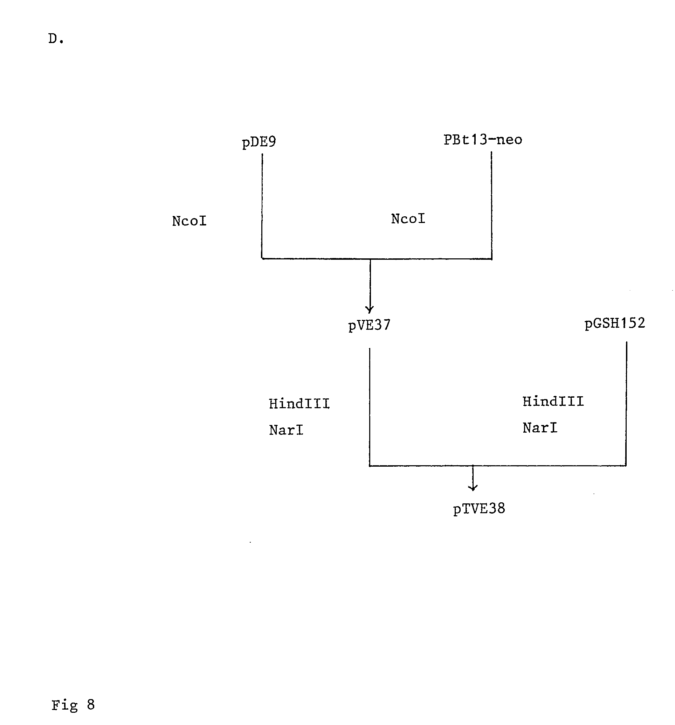

From the chimeric E. coli expression constructs pBt134, pBt135 and pBt13-neo, the bt13 gene was isolated and inserted in plant expression vectors. Cloning of the BamHI fragment from pBt135 in pGSH160 and pGSJ280 (Deblaere et al (1987) Methods in Enzymology 153, 277-292) yielded pGSJ141 and pGSJ142 respectively (Fig. 8A). These plasmids carried the bt13 gene under the control of the 35S promoter (Odell et al (1985)) and the TR2′ promoter (Velten and Schell (1985)), respectively. A kanamycin resistance gene was present as a selection marker for transformation. Similarly, a NcoI-BamHI fragment from pBt134 was isolated and was ligated in the same vectors, yielding the constructs pGSJ143 and pGSJ144 containing the bt13-57 gene under the control of the 35S and TR2′ promoters respectively (Fig. 8B). Also, the BamHI-BglII fragment from pBt130-neo, comprising the bt13-neo hybrid gene, was cloned in pGSH150 and pGSJ270 (Deblaere et al (1987)), yielding pGSJ147 and pGSJ148 with the bt13-neo hybrid gene under the control of the TR2′ or 35S promoter (Fig. 8C). In the latter plasmids, no selection marker gene was present, since transformed plants could be directly selected for expression of the fusion protein.

In a similar way (Fig. 8D), the bt13-neo hybrid gene was placed under the control of a 35S3 promoter (Hull and Howell (1987)). The 35S3 promotor was cloned in pUC18 (Yanish-Perron et al (1985)), yielding pDE9, which contained an NcoI site at the first ATG codon in the 35S3 transcript. The 3′ terminal-NcoI site of the promoter fragment was created at the first ATG codon by site directed mutagenesis (Stanssens et al (1987)). A NcoI fragment containing the bt13 gene fragment and part of the neo gene was isolated from pBt13-neo and cloned in the NcoI site of pDE9, yielding pVE37. From pVE37, a HindIII-NarI fragment containing the 35S3 promotor-bt13-neo chimeric construct was isolated and cloned in pGSH152 digested with HindIII and NarI. This yielded pTVE38, which contained the bt13-neo hybrid gene under the control of the 35S3 promotor and was followed by the 3′ untranslated end of the T-DNA gene 7. This chimeric construct was located between the T-DNA border sequences.

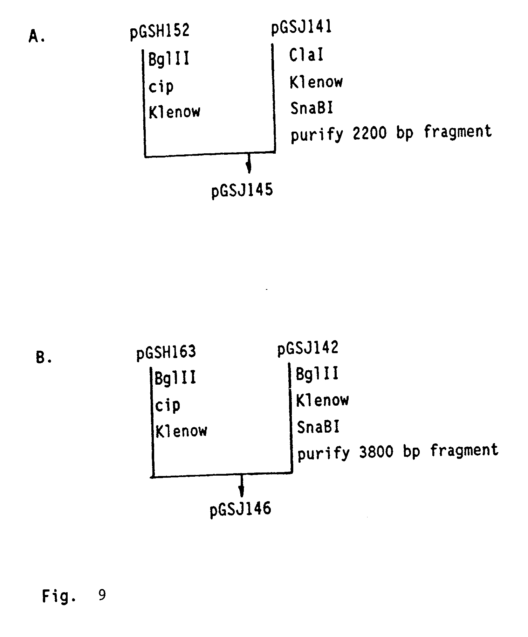

To express the bt13 and bt2 genes simultaneously in plants, each gene was placed under the control of its own strong constitutive promoter (Fig. 9). In this regard, A ClaI-SnaBI fragment was isolated from pGSJ141 and introduced in pGSH152 (U.S. patent application 821,582; Vaeck et al (1987)). This yielded pGSJ145 carrying the bt13 gene under the control of the TR1′ promoter (Velten and Schell (1985)) and the bt-neo-860 hybrid gene under the control of the TR2′ promoter (Velten and Schell (1985)). The bt-neo-860 gene is a fusion of the bt2 gene and the neo gene described in Example 9 of U.S. patent application 821,582.

Insertion of a BglII-SnaBI fragment from pGSJ142 into pGSH163 (U.S. patent application 821,582; Vaeck et al (1987)) yielded pGSJ146. pGSJ146 contained the bt13 gene under the control of a TR2′ promoter, the bt884 gene under the control of another TR2′ promoter, and the neo gene, as a selection marker, under the control of a TR1′ promoter. The bt884 gene is a truncated bt2 gene as described in Example 8 of U.S. patent application 821,582.

To form a hybrid of the bt13-57 gene, coding for just the Bt13 protein, and the neo gene (the "bt-57-neo gene") that can be expressed in plants, a plant expression construction pTVE39 was also made, using the same procedures as described above for constructing pTVE38.

## Example 6 - Transformation of tobacco, potato and tomato plants

The plant expression constructs from Example 5 were transferred from E. coli into Agrobacterium tumefaciens strain C58C1Rif carrying the plasmid pGV2260 (Vaeck et al (1987); De Blaere et al (1985) NAR 13, 4777 et seq). The pGV2260 provides the vir gene functions required for transfer of the T-DNA region to the plant genome.

Mobilization of the plant expression constructs was carried out using E. coli HB101 containing pRK2013 (Figurski et al (1979) PNAS 76, 1648 et seq) as a helper (European patent publication 0,116,718). The resulting recombined Agrobacterium strains were then used to transform potato plants (Solanium tuberosum cv. Berolina, Bintje and Désirée) by means of tuber disc infection (De Block et al (1987) EMBO J. 6, 2513-2518). Calli were selected on 50 mg/l kanamycin, and resistant calli were used for shoot induction. Elongated shoots were separated and transferred to rooting medium. Rooted shoots were further propagated to regenerate plants. Total DNA, prepared from cells of these plants (Dellaporta et al (1984) Plant Molecular Biology Reporter 1, 19-21), showed the presence of the chimeric genes by hybridization with a labelled bt13 gene fragment.

Tobacco and tomato plants were similarly transformed with Agrobacterium tumefaciens and selected on kanamycin according to the methods described in European patent application 87/400141.5.

## Example 7 - Expression of the bt13-neo and bt13-57-neo hybrid genes in transformed potato and tobacco plants

Calli of the potato and tobacco plants from Example 6, transformed with pTVE38 and pTVE39, were tested for NPTII activity by in situ phosphorylation assay using $^{32}$P-ATP (Reiss et al (1984) Gene 30, 217-223; U.S. patent application 821,582). Significant NPTII activity was detected in calli of the transformed plants. Previously, the transformed calli of tobacco and potato plants had been selected on a medium containing 50 mg/l kanamycin.

These tests demonstrated that the cells of the transformed calli expressed the hybrid genes, with which they had been transformed. Such expression involved expression of the full bt13 gene and the bt-57 gene coding for just the Bt13 protein.

Example 8 - Effect of the expression of the bt13 gene and fragments of the bt13 gene in transformed potato and tomato plants on Leptinotarsa decemlineata (Colorado potato beetle)

Larvae of L. decemlineata are fed with either potato leaves or tomato leaves in plastic containers. After 25 days, the larvae are transferred to containers filled with wet sand. The larvae dig in the sand and pupate. A week later, the adults emerge. They are placed in rearing cages with plant leaves. The eggs, laid on the leaves, are collected daily. After 4 to 5 days, the eggs hatch. The entire life cycle of the beetles occurs in a controlled environment cupboard maintained at 25°C, 70% relative humidity, and 18:6 Light: Dark photoperiod.

The killing of neonate larvae shows the toxicity of the Bt13 protein. Each larva is kept separately on a 2% agar medium and is supplied with a disc (0.28 cm$^2$) that is cut from either:

1) a fresh tomato or potato leaf to which is applied 5 μl of an aqueous solution of the Bt13 protein from the transformed E. coli K-12ΔH1Δtrp of Examples 1 and 2; or

2) a fresh leaf from a transformed tomato or potato plant of Example 6. After the leaf disc is consumed, fresh pieces of untreated leaf from untransformed tomato or potato plants are supplied for the next five days. Significantly more larvae are found dead over the five days of the test among the larvae who consume the treated leaf discs and the leaf discs from transformed plants than among the control larvae who consume only untreated leaves from untransformed plants.

Needless to say, this invention is not limited to the use of the bt13 gene. Rather, the invention also encompasses the use of the DNA sequence of Fig. 2, starting at position 737 and ending at position 2497, which codes for the pure Bt13 protein, as well as other DNA sequences encoding proteins that are the same as or equivalent to the Bt13 protein. The invention further relates to all DNA recombinants including the bt13 gene or a fragment thereof suitable for the transformation of a plant cell.

This invention also is not limited to transgenic potato and tomato plants that express the bt13 gene and are resistant to Coleoptera. It also relates to any plant which can be transformed in a like manner such as oilseed rape, alfalfa, sunflower, cotton, celery, onion, cloves, corn, soybean, tobacco, brassicas and sugarbeet.

## Claims

1. A transformed plant cell, characterized by a bt13 gene or a fragment thereof that codes for at least a 66 kDa protein and that is inserted in the genome of the cell.

2. The cell of claim 2, in which the bt13 gene fragment is inserted in the genome; the fragment having a DNA sequence as shown in Fig. 2, starting from position 737 and ending at position 2497 and coding for a Coleoptera toxin that consists essentially of a 66 kDa protein.

3. The cell of claim 1 or 2, in which the bt13 gene is downstream of, and under the control of, a strong constitutive plant promoter which is a 35S promoter, a TR1' promoter, or a TR2' promoter.

4. The cell of anyone of claims 1 to 3, in which the bt13 gene is upstream of polyadenylation and transcription termination signals which are from the octopine synthase gene or the T-DNA gene 7.

5. The cell of anyone of claims 1 to 4, in which the bt13 gene is in the same transcriptional unit as, and under the control of the same promoter as, a selectable marker gene.

6. The cell of anyone of claims 1 to 5 in which a gene, which codes for a Lepidoptera toxin, is also inserted in the genome of the cell.

7. A plant in which is the transformed cell of anyone of claims 1 to 6.

8. A seed of a plant of claim 7.

9. A method for rendering a plant resistant to Coleoptera characterized by: providing the plant with the transformed cell of anyone of claims 1 to 6.

10. DNA characterized by the DNA sequence shown in Figure 2, starting from position 737 and ending at position 2497,

11. A 66 kDa crystal protein encode by the DNA sequence of claim 10.

12. A microorganism, particularly E. coli, transformed with the DNA sequence of claim 10.

FIG.1

Bt 13 GENE

HindIII  XmnI  PstI  BalI  NdeI  MstII EcoRI  XbaI AccI EcoRV EcoRI  XmnI  SspI AccI SspI BglII HindIII

100bp

EP 0 305 275 A2

FIG 2

```
CTTAATTAAAGATAATATCTTTGAATTGTAACGCCCCTCAAAAGTAAGAACTACAAAAAAAGAATACGTTATATAGAAATATGTTTGAAC
        10        20        30        40        50        60        70        80        90

CTTCTTCAGATTACAAATATATTCGGACGGACTCTACCTCAAATGCTTATCTAACTATAGAATGACATACAAGCACAACCTTGAAAATTT
        100       110       120       130       140       150       160       170       180

GAAAATATAACTACCAATGAACTTGTTCATGTGAATTATCGCTGTATTTAATTTTCTCAATTCAATATATAATATGCCAATACATTGTTA
        190       200       210       220       230       240       250       260       270

CAAGTAGAAATTAAGACACCCTTGATAGCCTTACTATACCTAACATGATGTAGTATTAAATGAATATGTAAATATATTTATGATAAGAAG
        280       290       300       310       320       330         340       350       360

CGACTTATTTATAATCATTACATATTTTTCTATTGGAATGATTAAGATTCCAATAGAATAGTGTATAAATTATTTATCTTGAAAGGAGGG
        370       380       390       400       410       420        430       440       450

ATGCCTAAAAAACGAAGAACATTAAAAACATATATTTGCACCGTCTAATGGATTTATGAAAAATCATTTTATCAGTTTGAAAATTATGTAT
        460       470       480       490       500       510       520       530       540
```

```
                        MetAsnProAsnAsnArgSerGluHisAspThrIleLysThrThrGluAsnAsnGluValProThr
TATGATAAGAAAGGGAGGAAGAAAAATGAATCCGAACAATCGAAGTGAACATGATACAATAAAAACTACTGAAAATAATGAGGTGCCAAC
        550       560       570 ·     580       590       600       610       620       630
```

```
AsnHisValGlnTyrProLeuAlaGluThrProAsnProThrLeuGluAspLeuAsnTyrLysGluPheLeuArgMetThrAlaAspAsn
TAACCATGTTCAATATCCTTTAGCGGAAACTCCAAATCCAACACTAGAAGATTTAAATTATAAAGAGTTTTTAAGAATGACTGCAGATAA
        640       650       660       670       680       690       700       710       720
```

```
AsnThrGluAlaLeuAspSerSerThrThrLysAspValIleGlnLysGlyIleSerValValGlyAspLeuLeuGlyValValGlyPhe
TAATACGGAAGCACTAGATAGCTCTACAACAAAAGATGTCATTCAAAAAGGCATTTCCGTAGTAGGTGATCTCCTAGGCGTAGTAGGTTT
        730            SerSerProThrAspLysGlnValIleGlnLysGlyIleSerValValGlyAspLeuLeuGlyVal        810
                       ‾‾‾ ‾‾‾ ‾‾‾ ‾‾‾ ‾‾‾
                       Asp   Ser   ?     Asp
                            5'- CCTACTGATAAAGACGTTATTCAAAAAGG - 3'
ProPheGlyGlyAlaLeuValSerPheTyrThrAsnPheLeuAsnThrIleTrpProSerGluAspProTrpLysAlaPheMetGluGln
CCCGTTTGGTGGAGCGCTTGTTTCGTTTTATACAAACTTTTTAAATACTATTTGGCCAAGTGAAGACCCGTGGAAGGCTTTTATGGAACA
        820       830       840       850       860       870       880       890       900
```

```
ValGluAlaLeuMetAspGlnLysIleAlaAspTyrAlaLysAsnLysAlaLeuAlaGluLeuGlnGlyLeuGlnAsnAsnValGluAsp
AGTAGAAGCATTGATGGATCAGAAAATAGCTGATTATGCAAAAAAATAAAGCTCTTGCAGAGTTACAGGGGCCTTCAAAATAATGTCGAAGA
        910       920       930       940       950       960       970       980       990
```

```
TyrValSerAlaLeuSerSerTrpGlnLysAsnProValSerSerArgAsnProHisSerGlnGlyArgIleArgGluLeuPheSerGln
TTATGTGAGTGCATTGAGTTCATGGCAAAAAAAATCCTGTGAGTTCACGAAATCCACATAGCCAGGGGCGGATAAGAGAGCTGTTTTCTCA
        1000      1010      1020      1030      1040      1050      1060      1070      1080
```

```
AlaGluSerHisPheArgAsnSerMetProSerPheAlaIleSerGlyTyrGluValLeuPheLeuThrThrTyrAlaGlnAlaAlaAsn
AGCAGAAAGTCATTTTCGTAATTCAATGCCTTCGTTTGCAATTTCTGGATACGAGGTTCTATTTCTAACAACATATGCACAAGCTGCCAA
        1090      1100      1110      1120      1130      1140      1150      1160      1170
```

```
ThrHisLeuPheLeuLeuLysAspAlaGlnIleTyrGlyGluGluTrpGlyTyrGluLysGluAspIleAlaGluPheTyrLysArgGln
CACACATTTATTTTTACTAAAAGACGCTCAAATTTATGGAGAAGAATGGGGATACGAAAAAGAAGATATTGCTGAATTTTATAAAAGACA
        1180      1190      1200      1210      1220      1230      1240      1250      1260
```

```
LeuLysLeuThrGlnGluTyrThrAspHisCysValLysTrpTyrAsnValGlyLeuAspLysLeuArgGlySerSerTyrGluSerTrp
ACTAAAACTTACGCAAGAATATACTGACCATTGTGTCAAATGGTATAATGTTGGATTAGATAAATTAAGAGGTTCATCTTATGAATCTTG

ValAsnPheAsnArgTyrArgArgGluMetThrLeuThrValLeuAspLeuIleAlaLeuPheProLeuTyrAspValArgLeuTyrPro
GGTAAACTTTAACCGTTATCGCAGAGAGATGACATTAACAGTATTAGATTTAATTGCACTATTTCCATTGTATGATGTTCGGCTATACCC

LysGluValLysThrGluLeuThrArgAspValLeuThrAspProIleValGlyValAsnAsnLeuArgGlyTyrGlyThrThrPheSer
AAAAGAAGTTAAAACCGAATTAACAAGAGACGTTTTAACAGATCCAATTGTCGGAGTCAACAACCTTAGGGGCTATGGAACAACCTTCTC

AsnIleGluAsnTyrIleArgLysProHisLeuPheAspTyrLeuHisArgIleGlnPheHisThrArgPheGlnProGlyTyrTyrGly
TAATATAGAAAATTATATTCGAAAACCACATCTATTTGACTATCTGCATAGAATTCAATTTCACACGCGGTTCCAACCAGGATATTATGG

AsnAspSerPheAsnTyrTrpSerGlyAsnTyrValSerThrArgProSerIleGlySerAsnAspIleIleThrSerProPheTyrGly
AAATGACTCTTTCAATTATTGGTCCGGTAATTATGTTTCAACTAGACCAAGCATAGGATCAAATGATATAATCACATCTCCATTCTATGG

AsnLysSerSerGluProValGlnAsnLeuGluPheAsnGlyGluLysValTyrArgAlaValAlaAsnThrAsnLeuAlaValTrpPro
AAATAAATCCAGTGAACCTGTACAAAATTTAGAATTTAATGGAGAAAAAGTCTATAGAGCCGTAGCAAATACAAATCTTGCGGTCTGGCC

SerAlaValTyrSerGlyValThrLysValGluPheSerGlnTyrAsnAspGlnThrAspGluAlaSerThrGlnThrTyrAspSerLys
GTCCGCTGTATATTCAGGTGTTACAAAAGTGGAATTTAGCCAATATAATGATCAAACAGATGAAGCAAGTACACAAACGTACGACTCAAA

ArgAsnValGlyAlaValSerTrpAspSerIleAspGlnLeuProProGluThrThrAspGluProLeuGluLysGlyTyrSerHisGln
AAGAAATGTTGGCGCGGTCAGCTGGGATTCTATCGATCAATTGCCTCCAGAAACAACAGATGAACCTCTAGAAAAGGGGATATAGCCATCA

LeuAsnTyrValMetCysPheLeuMetGlnGlySerArgGlyThrIleProValLeuThrTrpThrHisLysSerValAspPhePheAsn
ACTCAATTATGTAATGTGCTTTTTTAATGCAGGGTAGTAGAGGAACAATCCCAGTGTTAACTTGGACACATAAAAGTGTAGACTTTTTTAA

MetIleAspSerLysLysIleThrGlnLeuProLeuValLysAlaTyrLysLeuGlnSerGlyAlaSerValValAlaGlyProArgPhe
CATGATTGATTCGAAAAAAAATTACACAACTTCCGTTAGTAAAGGCATATAAGTTACAATCTGGTGCTTCCGTTGTCGCAGGTCCTAGGTT

ThrGlyGlyAspIleIleGlnCysThrGluAsnGlySerAlaAlaThrIleTyrValThrProAspValSerTyrSerGlnLysTyrArg
TACAGGAGGAGATATCATTCAATGCACAGAAAATGGAAGTGCGGCAACTATTTACGTTACACCGGATGTGTCGTACTCTCAAAAATATCG

AlaArgIleHisTyrAlaSerThrSerGlnIleThrPheThrLeuSerLeuAspGlyAlaProPheAsnGlnTyrTyrPheAspLysThr
AGCTAGAATTCATTATGCTTCTACATCTCAGATAACATTTACACTCAGTTTAGACGGGGCACCATTTAATCAATACTATTTCGATAAAAC

IleAsnLysGlyAspThrLeuThrTyrAsnSerPheAsnLeuAlaSerPheSerThrProPheGluLeuSerGlyAsnAsnLeuGlnIle
GATAAATAAAAGGAGACACATTAACGTATAATTCATTTAATTTAGCAAGTTTCAGCACACCATTCGAATTATCAGGGAATAACTTACAAAT
```

GlyValThrGlyLeuSerAlaGlyAspLysValTyrIleAspLysIleGluPheIleProValAsn
AGGCGTCACAGGATTAAGTGCTGGAGATAAAGTTTATATAGACAAAATTGAATTTATTCCAGTGAATTAAATTAACTAGAAAGTAAAGAA

GTAGTCACCATCTATGATAGTAAGCAAAGGATAAAAAAAATGAGTTCATAAAATGAATAACATAGTGTTCTTCAACTTTCGCTTTTTGAAG

GTAGATGAAGAACACTATTTTTATTTTCAAAATGAAGGAAGTTTTAAATATGTAATCATTTAAAGGGAACAATGAAAGTAGGAAATAAGT

CATTATCTATAACAAAATAACATTTTTATATAGCCAGAAATGAATTATAATATTAATCTTTTCTAAATTGACGTTTTTCTAAACGTTCTA

TAGCTTCAAGACGCTTAGAATCATCAATATTTGTATACAGAGCTGTTGTTTCCATCGAGTTATGTCCCATTTGATTCGCTAATAGAACAA

GATCTTTATTTTCGTTATAATGATTGGTTGCATAAGTATGGCGTAATTTATGACGGCTTTTCTTTTCATCAAAAGCCCTCGTGTATTTCT

CTGTAAGCTT

```
  C  T E N G S A A T I Y V T P D V S Y S Q K Y R A R I H Y A S T S Q I T F T L S L
AATGCACAGAAAATGGAAGTGCGGCAACTATTTACGTTACACCGGATGTGTCGTACTCTCAAAAATATCGAGCTAGAATTCATTATGCTTCTACATCTCAGATAACATTTACACTCAGTT
        10        20        30        40        50        60        70        80        90       100       110       120


  D  G A P F N Q Y Y F D K T I N K G D T L T Y N S F N L A S F S T P F E L S G N N
TAGACGGGGCACCATTTAATCAATACTATTTCGATAAAACGATAAATAAAGGAGACACATTAACGTATAATTCATTTAATTTAGCAAGTTTCAGCACACCATTCGAATTATCAGGGAATA
       130       140       150       160       170       180       190       200       210       220       230       240


  L  Q I G V T G L S A G D K │V│Y│I│D│K│I│E│F│I│P│V N *
ACTTACAAATAGGCGTCACAGGATTAAGTGCIGGAGATAAAGTTTATATAGACAAAATTGAATTTATTCCAGTGAATTAAATTAATCAGAAA
      250       260       270       280       290       300       310       320       330
```

| | | | | │V│ │Y│ │I│ │D│ │K│ │I│ │E│ │F│ │I│ │P│ | |
|---|---|---|---|---|---|---|---|---|---|---|---|---|---|---|
| Bt 2 | S | G | N | E | V | Y | I | D | R | I | E | F | V | P | A | E |
| Bt HD1 | | | | | | | | | | | | | | | | |
| Bt HD73 | G | T | A | G | V | I | I | D | R | F | E | F | I | P | V | T |
| Bt 4 | I | Q | S | G | V | Y | I | D | R | I | E | F | I | P | V | T |
| Bt 8 | S | N | N | Q | V | I | I | D | R | I | E | I | I | P | I | T |
| Bt syntro | H | S | Y | N | I | Y | I | D | K | I | E | F | I | P | I | T |
| | | | | | | | | | | | | | | | | |
| consensus: | | | | | V | I/Y | I | D | K/R | I | E | F | I | P | | |

FIG 3

FIG.4

HindIII    EcoRI    EcoRI    HindIII-EcoRI

bt13

1           2

pMA5-8         pBt130

EcoRI           EcoRI

HindIII        HindIII

1           2

pMABt131        pMABt135

```
                M    N    P    N
pMABt131     ATG'AAT'CCG'AAC'

                1    2    3    4

+ oligo 1


                M    D    P    N
pMCBt131     ATG'[G]AT'CCG'AAC'
                 BamHI
```

```
              N    T    E    A    L    D    S
pMABt131     AAT  ACG  GAA  GCA  CTA  GAT  AGC

                            55             59

+ oligo 2


                                       D    S
pMCBt133     A[GG] A[TG] GAA  GCA  CTA  GAT  AGC
             Fokl ─────────────────┘
```

```
              E    F    I    P    V    N    *
pMABt135     GAA  TTT  ATT  CCA  GTG  AAT  TAA  ATT  AAC

                  640             644

+ oligo 3


              E    F    I    P    V    N    *
pMCBt136     GAA  TTT  ATT  CCA  G[TT] A[AC] T[G]A  ATT  CAC
                                  HpaI      EcoRI
```

Fig. 5

A.  pMCBt136           pBt130              pUC19

     EcoRI                PstI                EcoRI/Klenow

     Klenow               Bsp1286             PstI

     Bsp1286

     purify 200           purify 1600

     bp fragment          bp fragment

pBt136

 

TAA

                                            BamHI, SmaI, KpnI, EcoRI

Pstı               pBt136

 

B.  pMCBt133           pJB66               pBT136

     FokI

     Klenow               Ncol                OxaNI

     OxaNI                Klenow              BamHI

     purify 800 bp frgm.  BamHI

pBt134

 

pMCBt131           pJB63               pBt136

     BamHI                                    BamHI

     PstI                 BamHI               PstI

     purify 150 bp frgm.  Cip                 purify 1600 bp frgm.

pBt135

Fig. 6

pBt135                 pLKM91

| partial HpaI | HindIII |
| BamHI | Klenow |
| | BglII |
| | purify fragment |

pBt13-neo

643             5

---GTT,AGC,TTG,GAT,GGA

<u>bt13</u>       <u>neo</u>

Fig. 7

A.

pBt135      pGSH160    pGSJ280

BamHI

BamHI
cip

BamHI
cip

pGSJ141

pGSJ142

B.

pBt134      pGSH160     pGSJ280

NcoI

Klenow        ClaI

BamHI        Klenow

purify         BamHI

fragment

pGSJ143

pGSJ144

C.

pBt13 neo        pGSH150     pGSJ270

BamHI

BglII              BamHI

purify              cip

pGSJ147

pGSJ148

Fig. 8

D.

Fig 8

A.
pGSH152      pGSJ141

| BglII | ClaI
| cip | Klenow
| Klenow | SnaBI
| | purify 2200 bp fragment

pGSJ145

B.
pGSH163      pGSJ142

| BglII | BglII
| cip | Klenow
| Klenow | SnaBI
| | purify 3800 bp fragment

pGSJ146

Fig. 9